# FASCICULE DE BREVET EUROPEEN

(11) **EP 3 796 953 B1**
(45) Date de publication et mention de la délivrance du brevet: **31.07.2024**
(21) Numéro de dépôt: 19740614.3
(22) Date de dépôt: 22.05.2019
(51) Int. Cl.: A61M 60/178, A61M 60/216, A61M 60/861, A61M 60/865

(54) **DISPOSITIF D'ANCRAGE D'UNE POMPE CARDIAQUE**
VORRICHTUNG ZUR VERANKERUNG EINER HERZPUMPE
DEVICE FOR ANCHORING A HEART PUMP

(30) Priorité: 22.05.2018 FR 1854245
(43) Date de publication de la demande: 31.03.2021
(73) Titulaire: Fineheart, 33600 Pessac (FR)
(72) Inventeur: COLLAS, Jérémy, 33200 Bordeaux (FR); ROUSSEAU, Jean-Baptiste, 33000 Bordeaux (FR); MASCARELL, Arnaud, 37250 Montbazon (FR); GARRIGUE, Stéphane, 33130 Begles (FR)
(74) Mandataire: Plasseraud IP
(86) Numéro de dépôt international: PCT/FR2019/051162
(87) Numéro de publication internationale: WO 2019/224478

(56) Documents cités:
- WO-A1-2012/018917
- US-A1- 2006 235 357
- US-A1- 2008 009 891
- US-A1- 2013 012 761
- US-A1- 2017 281 840

## Description

### ARRIERE-PLAN DE L'INVENTION

### Domaine de l'invention

La présente invention concerne un dispositif d'ancrage d'une pompe cardiaque dans une ouverture d'une paroi ventriculaire d'un coeur battant.

Elle concerne également un procédé (non revendiqué) pour la pose de ce dispositif d'ancrage sur une telle paroi.

### Arrière-plan technologique

L'insuffisance cardiaque (IC), est un état pathologique dans lequel le coeur d'un patient présente une incapacité à fournir un débit sanguin nécessaire aux besoins métaboliques de l'organisme.

Il est connu pour traiter l'insuffisance cardiaque d'implanter un dispositif d'assistance ventriculaire (DAV), qui est une pompe cardiaque artificielle.

Cette pompe mécanique ne remplace pas le coeur qui continue à fonctionner, mais apporte une aide au ventricule affaibli afin d'accroître le débit sanguin de façon adaptée aux besoins de l'individu.

Cette assistance peut être temporaire dans l'attente d'un greffon disponible pour réaliser une transplantation cardiaque.

Cependant, on observe une proportion significative de patients qui ne recevront pas un tel greffon, soit parce qu'ils ne peuvent être candidats à une telle transplantation, par exemple en raison d'une insuffisance cardiaque sévère, soit parce qu'aucun greffon adapté n'est disponible pour ces patients.

Dans ce cas, l'assistance ventriculaire est utilisée en destination, c'est-à-dire que la pompe cardiaque artificielle est implantée à long terme.

Ces pompes cardiaques font donc l'objet d'intenses recherches visant à améliorer la survie et la qualité de vie des patients présentant une insuffisance cardiaque.

De nombreuses avancées ont été réalisées ces dernières années et on connaît aujourd'hui des dispositifs d'assistance ventriculaire plus compacts, silencieux et présentant une durée de service accrue.

Les pompes cardiaques implantables de l'état de l'art sont ainsi typiquement équipées d'un moteur électrique intégré pour assurer leur fonctionnement, la vitesse de rotation de la pompe fournissant la force nécessaire pour faire circuler le sang depuis le ventricule affaibli vers la circulation corporelle.

On connaît des systèmes d'implantation de telles pompes dans une ouverture d'une paroi ventriculaire.

Ces systèmes d'implantation comprennent généralement une portion tubulaire aux extrémités de laquelle sont placés, ou formés, des collerettes destinées à être plaquées chacune contre une face opposée de la paroi ventriculaire après introduction de la portion tubulaire dans une ouverture réalisée dans cette paroi ventriculaire avec un dispositif de carottage.

Ces collerettes permettent ainsi de maintenir en position cette portion tubulaire creuse, laquelle définit alors un conduit ouvert traversant la paroi ventriculaire.

A l'extrémité, placée à l'extérieur du coeur, de cette portion tubulaire, est introduite une pompe cardiaque qui une fois installée, assure le renvoi du sang présent dans le ventricule vers la circulation corporelle.

Bien que représentant un progrès certain pour la qualité de vie d'un patient souffrant d'insuffisance cardiaque, de nombreux inconvénients sont encore constatés.

Notamment, la réalisation de cette ouverture sur un coeur battant, pour minimiser les effets secondaires de l'intervention sur le patient, peut entraîner des pertes de sang importantes durant l'implantation de la pompe cardiaque.

Une grande dextérité et une rapidité d'action des praticiens sont en conséquence, des facteurs importants de réussite, ce qui est source de stress pour l'équipe réalisant l'intervention.

En outre, il est souhaitable que l'extrémité d'éjection du sang de la pompe soit centrée sur la valve cardiaque pour assurer un flux de sang éjecté optimal.

Le positionnement de l'ouverture dans la paroi ventriculaire participe alors des performances réelles de la pompe cardiaque dans l'éjection du sang dans l'aorte.

Or, on constate qu'en l'absence de repères, cette ouverture n'est pas toujours bien positionnée au regard de la valve cardiaque. Le flux de sang n'est ainsi pas idéalement éjecté.

Il existe donc un besoin pressant pour un dispositif d'ancrage dont la conception originale surmonte les inconvénients décrits ci-dessus.

US2006235357A1 décrit une canule pour le transport du sang, reliant le coeur avec une pompe cardiaque.

### Objet de l'invention

La présente invention vise à pallier les inconvénients de l'art antérieur et à répondre aux contraintes ci-dessus énoncées en proposant un dispositif d'ancrage d'une pompe cardiaque et son procédé de pose, simple dans leur conception et dans leur mode opératoire, fiable et assurant une étanchéité totale entre l'intérieur et l'extérieur du coeur.

Un autre objet de la présente invention est un tel dispositif d'ancrage et un tel procédé réduisant la complexité de l'intervention chirurgicale.

Un autre objet de la présente invention est un tel dispositif d'ancrage et un tel procédé permettant de réduire les pertes de sang pour le patient lors de l'intervention chirurgicale et d'accroître sa récupération.

### BREVE DESCRIPTION DE L'INVENTION

A cet effet, l'invention concerne un procédé de pose d'un dispositif d'ancrage d'une pompe cardiaque sur un coeur.

Selon l'invention, ledit dispositif d'ancrage comprenant un corps creux à l'extrémité proximale duquel est destinée à être placée une première bride rapportée et à l'extrémité distale duquel est placée une membrane auto-expansible entre une première configuration, dite déformée, dans laquelle ladite membrane présente une forme tubulaire, ou sensiblement tubulaire, et une seconde configuration, dite initiale, dans laquelle elle définit une seconde bride, on réalise les étapes suivantes :
a) ladite première bride étant distincte de l'ensemble corps creux/membrane auto-expansible, assembler d'abord ladite première bride à la surface externe de la paroi ventriculaire dudit coeur,
b) ladite première bride comportant un trou, de préférence central, ladite première bride est agencée de sorte que ledit trou communique avec une ouverture dans la paroi ventriculaire lorsque cette dernière existe déjà, ou réaliser une ouverture dans ladite paroi ventriculaire au travers dudit trou de ladite première bride ainsi assemblée, lorsque cette ouverture n'est pas encore formée,
c) introduire au travers dudit trou de ladite première bride et de ladite ouverture dans la paroi ventriculaire, l'ensemble comprenant ledit corps creux/membrane auto-expansible dans sa configuration déformée,
d) ladite membrane auto-expansible étant placée dans la chambre ventriculaire et prenant sa configuration initiale, plaquer ladite seconde bride contre une surface intérieure de ladite paroi ventriculaire, ledit corps creux étant configuré pour que son extrémité proximale forme alors saillie de ladite première bride à l'extérieur dudit coeur,
e) assembler de manière étanche ladite première bride et ledit corps creux de sorte que lesdites brides étant plaquées de part et d'autre de ladite paroi ventriculaire, ledit dispositif d'ancrage est maintenu, ou verrouillé, en position.

Un tel dispositif d'ancrage d'une pompe cardiaque dans l'ouverture d'une paroi ventriculaire d'un coeur battant est encore connu sous le nom de bague d'ancrage, ou de dispositif de fixation, d'une pompe cardiaque.

De manière avantageuse, un tel dispositif d'ancrage assure un confort accru pour l'équipe chirurgicale lors de l'intervention car ce dispositif d'ancrage est d'une pose plus aisée. Il assure également une sécurité renforcée pour le patient car une étanchéité totale est réalisée entre l'intérieur et l'extérieur du coeur du patient.

De manière avantageuse, la membrane auto-expansible présente une première configuration, dite déformée, dans laquelle elle présente une forme tubulaire, ou essentiellement tubulaire, et une seconde configuration, dite initiale, dans laquelle elle définit une bride, ou collerette, s'étendant radialement, ou sensiblement radialement, à partir dudit corps creux, cette bride étant destinée à venir en aboutement contre une face intérieure de ladite paroi ventriculaire du coeur battant

De préférence, l'ensemble corps creux/membrane auto-expansible comporte un élément d'étanchéité comprenant une valve anti-reflux, qui lors par exemple, du retrait de la pompe cardiaque reçue dans le corps creux se referme et redevient étanche. A titre d'exemple, il s'agit d'une membrane auto réparatrice, dite encore membrane auto-seal, intégrée dans le canal interne délimité par le corps creux de la bague d'ancrage. Cet élément d'étanchéité empêche avantageusement toute fuite de sang par ce canal interne.

Dans différents modes de réalisation particuliers de ce procédé de pose, chacun ayant ses avantages particuliers et susceptibles de nombreuses combinaisons techniques possibles:
- à l'étape a), on assemble ladite première bride par couture avec la surface externe de ladite paroi ventriculaire.
   Cette étape peut, par exemple, consister en la réalisation de plusieurs points de suture chirurgicale.
- à l'étape a), on assemble ladite première bride avec la surface externe de ladite paroi ventriculaire par aspiration.

De préférence, cette première bride comportant deux faces d'extrémités reliées entre elles par une paroi latérale et ladite face d'extrémité destinée à venir en aboutement contre la surface externe de ladite paroi ventriculaire, comprenant une rainure en communication avec un orifice débouchant latéralement, on plaque ladite face d'extrémité contre ladite surface externe et on crée un vide par aspiration pour assembler ladite première bride et ladite paroi ventriculaire.
- à l'étape a), ladite ouverture étant déjà présente dans ladite paroi ventriculaire, on centre le trou de ladite première bride sur ladite ouverture avant son assemblage avec ladite paroi ventriculaire.

On s'assure ainsi d'une optimisation des dimensions du chemin créé par le trou de ladite première bride et l'ouverture de ladite paroi ventriculaire.

Bien entendu, lorsque l'ouverture dans la paroi ventriculaire existe déjà, on agence le trou de ladite première bride de sorte qu'il communique et encore mieux, qu'il communique entièrement, avec cette ouverture, avant assemblage de cette première bride avec ladite paroi ventriculaire.
- préalablement à l'étape a), on réalise une incision dans la paroi ventriculaire et on introduit un câble de guidage dans la chambre ventriculaire de ce coeur et au travers de la valve cardiaque correspondante pour positionner ladite première bride.

Ce câble va non seulement guider la première bride en position mais est aussi destiné au guidage, notamment l'orientation, de la tête de carottage lors de son approche de la paroi ventriculaire en vue de la réalisation d'une ouverture par carottage dans cette paroi lorsque celle-ci n'est pas déjà formée.
- à l'étape e), on assemble ladite première bride et l'extrémité proximale dudit corps creux grâce à un moyen de serrage tel qu'un écrou ou une bague de serrage.

De préférence, ce moyen de serrage vient en appui contre l'autre face d'extrémité de la première bride.

Avantageusement, un élément d'étanchéité est placé entre ce moyen de serrage et cette première bride, tel qu'un joint torique.
- on introduit une pompe cardiaque dans le canal interne délimité par le corps creux du dispositif d'ancrage et on assemble cette pompe au corps creux.

De préférence, cet assemblage est un couplage temporaire, ou encore réversible, de sorte que la pompe cardiaque puisse être aisément retirée lors d'une étape de manutention.
- on réalise les différentes étapes de ce procédé sur un coeur battant.

La présente invention concerne encore un dispositif médical d'ancrage d'une pompe cardiaque dans une ouverture d'une paroi ventriculaire d'un coeur, comprenant :
- une première bride comprenant un trou, de préférence central,
- un ensemble comportant un corps creux à l'extrémité distale duquel est placé une membrane auto-expansible entre une première configuration, dite déformée, dans laquelle ladite membrane présente une forme tubulaire et une seconde configuration, dite initiale, dans laquelle elle définit une seconde bride,
- ladite première bride étant distincte dudit ensemble, ledit ensemble étant configuré pour être introduit au moins en partie au travers du trou de ladite première bride,
- un moyen de serrage pour assembler ladite première bride et l'extrémité proximale dudit corps creux lorsque cet ensemble a été inséré en partie dans le trou de ladite première bride, ledit moyen de serrage étant configuré pour coulisser le long dudit corps creux de manière à être plaqué contre ladite première bride.

Il ressort clairement que le moyen de serrage entoure la surface externe dudit corps creux lorsqu'il est engagé sur ce dernier.

De manière avantageuse, ce dispositif médical d'ancrage est réalisé entièrement dans un matériau biocompatible, non toxique et stérile.

Selon un mode de réalisation du dispositif médical d'ancrage de l'invention, le moyen de serrage est configuré pour réaliser un couplage réversible, ou temporaire, de ladite première bride et de l'extrémité proximale dudit corps creux.

Par « réversible », on entend que ladite première bride et ledit corps creux, après avoir été assemblés, peuvent être désassemblés l'un de l'autre tout en restant fonctionnels pour un nouvel assemblage ultérieur.

Selon un autre mode de réalisation du dispositif d'ancrage de l'invention, ce dispositif comprend également un premier élément d'étanchéité destiné à être placé entre ledit moyen de serrage et ladite première bride.

Cet élément d'étanchéité est avantageusement un joint torique.

De manière avantageuse, le moyen de serrage peut comprendre un logement pour recevoir ce premier élément d'étanchéité, ce dernier formant saillie du moyen de serrage lorsqu'il est placé dans son logement.

Selon encore un autre mode de réalisation de ce dispositif d'ancrage, il comporte un second élément d'étanchéité comprenant une valve anti-reflux placée dans le canal interne délimité par ledit corps creux.

A titre d'exemple, il s'agit d'une membrane auto réparatrice, dite encore membrane auto-seal, intégrée dans le canal interne délimité par le corps creux de la bague d'ancrage. Cette valve anti-reflux empêche toute fuite de sang par ce canal interne. Elle permet ainsi, après retrait du dispositif de carottage, de rétablir l'étanchéité lors de la pose du dispositif d'ancrage sur le coeur. Lors du retrait de la pompe cardiaque du coeur, par exemple pour une manutention, reçue dans le corps creux, ce second élément d'étanchéité se referme et redevient étanche.

A titre purement illustratif, cette valve anti-reflux est en silicone.

Selon encore un autre mode de réalisation de ce dispositif d'ancrage, cette première bride est réalisée dans un matériau suturable ou agrafable.

A titre purement illustratif, cette première bride est réalisée en Dacron^{®}. Alternativement, cette première bride est configurée pour être assemblée par réalisation d'une dépression entre ladite première bride et ladite paroi ventriculaire avec ladite paroi ventriculaire.

A titre d'exemple, cette première bride comportant deux faces d'extrémités reliées entre elles par une paroi latérale, sa face d'extrémité destinée à venir en aboutement contre la surface externe de ladite paroi ventriculaire, comprend une rainure circulaire en communication avec un orifice débouchant sur sa paroi latérale.

Selon encore un autre mode de réalisation de ce dispositif d'ancrage, ce corps creux a une longueur supérieure à la somme de l'épaisseur de la paroi ventriculaire et de la première bride.

Selon encore un autre mode de réalisation de ce dispositif d'ancrage, le moyen de serrage est un écrou.

Cet écrou présentant un filetage interne, la surface externe d'une partie du corps creux, en particulier son extrémité proximale, comporte un filetage complémentaire du filetage de l'écrou pour assurer l'engagement et le déplacement de l'écrou le long de ladite partie de surface externe dudit corps creux.

Selon encore un autre mode de réalisation de ce dispositif d'ancrage, l'extrémité proximale du corps creux comporte un alésage pour le vissage ou le passage d'une fixation telle qu'une vis, de manière à bloquer en position une pompe cardiaque introduite dans ce corps creux.

Selon encore un autre mode de réalisation de ce dispositif d'ancrage, ladite membrane auto-expansible est réalisée en nitinol.

### BREVE DESCRIPTION DES DESSINS

D'autres avantages, buts et caractéristiques particulières de la présente invention ressortiront de la description qui va suivre, faite, dans un but explicatif et nullement limitatif, en regard des dessins annexés, dans lesquels:
- la Figure 1 représente schématiquement un dispositif de carottage pour réaliser une ouverture dans une paroi ventriculaire d'un coeur battant selon un mode de réalisation particulier de la présente invention ;
- la Figure 2 montre le dispositif de carottage de la Fig. 1, un accessoire d'assistance à la pose d'une bague d'ancrage étant reçu à l'extrémité distale de ce dernier pour définir un ensemble de carottage et d'introduction d'une partie d'une bague d'ancrage d'un dispositif d'assistance ventriculaire ;
- la Figure 3 est une représentation schématique de l'ensemble de la Fig. 2, la tête de carottage étant dans sa position actionnée pour son introduction dans un petit orifice, préalablement réalisé avec un outil coupant dans la paroi ventriculaire du coeur ;
- la Figure 4 est une vue en perspective et en coupe de l'ensemble de la Fig. 2 juste après réalisation de la découpe dans la paroi ventriculaire ;
- la Figure 5 est une vue partielle et de dessus de l'ensemble de la Fig. 2, lors de l'assemblage de l'accessoire d'assistance avec la bride préalablement assemblée à la surface extérieure de ladite paroi ventriculaire, l'extrémité distale de l'accessoire d'assistance étant alors insérée dans l'ouverture réalisée ;
- la Figure 6 est une vue en coupe longitudinale de l'ensemble de la Fig. 2 et de l'apex du coeur, le dispositif de carottage étant en cours de retrait, l'extrémité distale de l'accessoire d'assistance, insérée dans l'ouverture réalisée, étant apparent ;

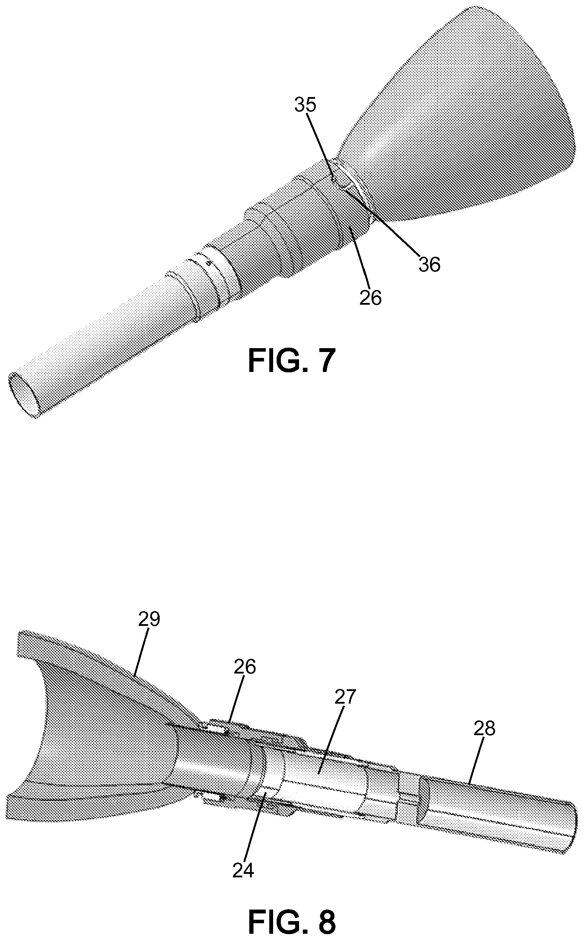

- la Figure 7 est une vue en perspective de l'accessoire d'assistance de la Fig. 2 assemblé à une bride solidaire de l'apex du coeur, une partie d'une bague d'ancrage comportant une bride dans sa configuration déformée étant introduite dans cet accessoire d'assistance au moyen d'un dispositif de poussée ;
- la Figure 8 est une vue en coupe longitudinale des éléments illustrés à la Fig. 7 ;

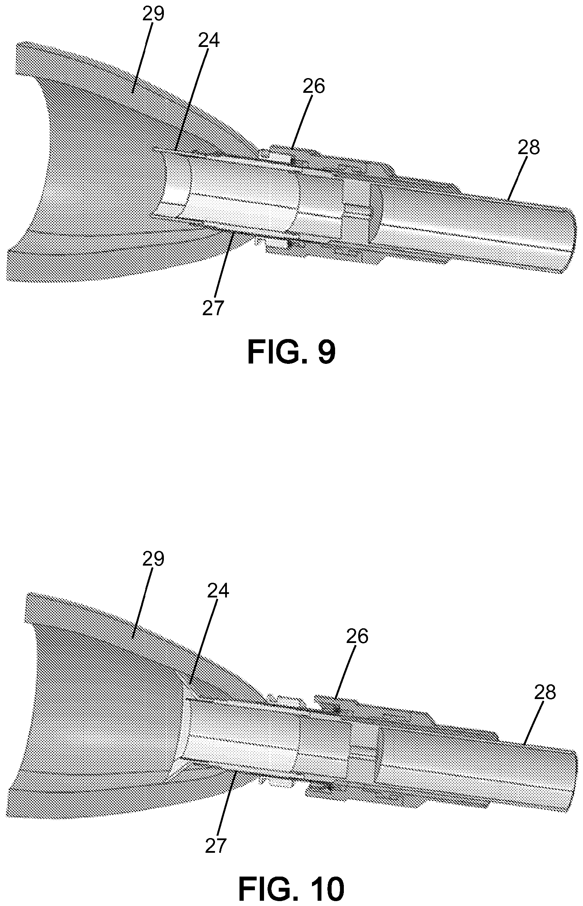

- la Figure 9 est une vue en coupe longitudinale des éléments illustrés à la Fig. 7, la bride dans sa configuration déformée étant introduite dans la chambre ventriculaire au moyen du dispositif de poussée ;
- la Figure 10 est une vue en coupe longitudinale des éléments illustrés à la Fig. 7, la bride étant dans sa configuration initiale pour définir une collerette, l'accessoire d'assistance à la pose ayant été désassemblé de la bride montée à la surface extérieure de la paroi ventriculaire et étant en cours de retrait ;
- la Figure 11 est une vue en perspective des éléments illustrés à la Fig. 7, l'accessoire d'assistance ayant été retiré, un joint d'étanchéité et un écrou étant engagés sur le dispositif de poussée pour bloquer en position de la bague d'ancrage ;
- la Figure 12 est une vue en perspective de la bague d'ancrage de la Fig. 11 montée dans son ouverture et supportant une pompe cardiaque.

### DESCRIPTION DETAILLEE DE MODE DE REALISATION DE L'INVENTION

Tout d'abord, on note que les figures ne sont pas à l'échelle.

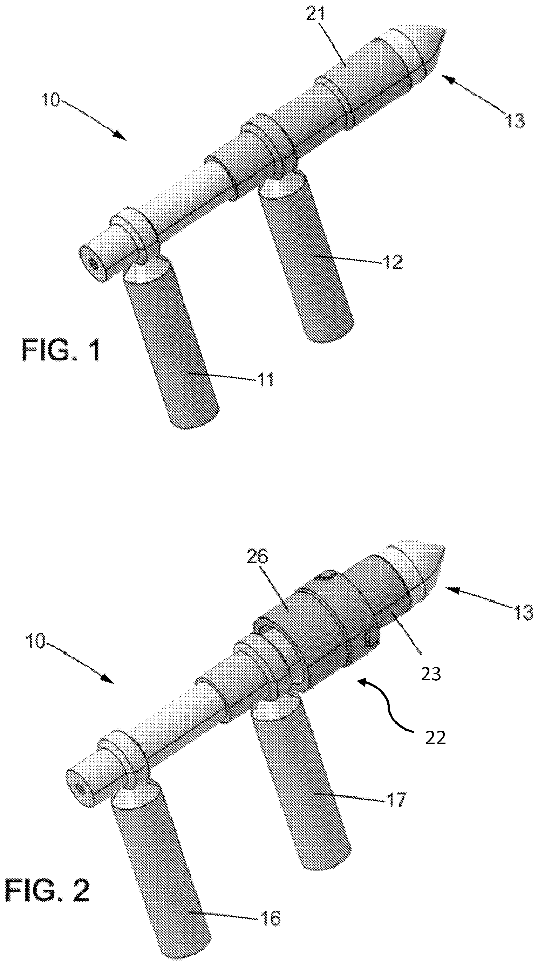

Les Figures 1 à 6 montrent de manière schématique un dispositif de carottage 10 pour réaliser une ouverture dans la paroi ventriculaire d'un coeur battant, selon un mode de réalisation particulier de la présente invention.

Ce dispositif de carottage 10 comporte deux parties de corps 11, 12 qui sont emboîtées l'une dans l'autre de sorte qu'une de ces parties est montée en coulissement dans l'autre partie de corps. Ce dispositif présente une extrémité proximale et une extrémité distale.

Dans le contexte de la présente invention, le terme « proximal » signifie la position la plus proche du professionnel de santé, ou du praticien, tandis que le terme « distal » doit ici être entendu comme signifiant le plus éloigné de ce professionnel. En d'autres termes, l'extrémité distale d'une pièce est l'extrémité qui serait la première engagée dans le coeur battant tandis que son extrémité proximale serait la dernière extrémité à y être engagée.

Ce dispositif de carottage 10 comprend une tête de carottage 13 comportant une pointe ayant une forme frustro-conique, à son extrémité distale, pour faciliter son insertion au travers d'une incision réalisée avec un outil coupant tel qu'un scalpel, dans la paroi ventriculaire de ce coeur battant. Cette tête 13 comporte également une lame de transsection 14 placée à son extrémité proximale. Cette lame de transsection 14 présente une section transverse droite de forme circulaire mais pourrait présenter d'autres formes appropriées telles qu'ovale.

Cette tête de carottage 13 est mobile entre une position de repos, dans laquelle la lame de transsection 14 est plaquée contre, ou est placée à proximité de, l'extrémité distale du corps principal du dispositif de carottage 10, et une position actionnée dans laquelle la lame de transsection 14 est placée à distance de l'extrémité distale du corps principal du dispositif de carottage 10.

Plus précisément, cette tête de carottage 13 est actionnée par un mécanisme d'actionnement comprenant une tige d'actionnement 15, à l'extrémité de laquelle cette tête 13 est montée, cette tige 15 étant couplée à un logement interne du corps principal du dispositif de carottage 10.

Ce corps principal comporte ici deux parties 11, 12 de corps emboîtées l'une dans l'autre, une première partie de corps étant mobile en translation par rapport à l'autre partie de corps de sorte que son déplacement dans et hors de l'autre partie de corps provoque le déplacement en translation de ladite tête de carottage 13.

Chaque partie de corps comporte une poignée 16, 17 de préhension ayant un axe longitudinal Z. Ces poignées 16, 17 sont ici arrangées de sorte que leur axe longitudinal est perpendiculaire à l'axe longitudinal défini par le corps du dispositif de carottage 10.

Ainsi, le praticien peut tenir d'une première main, une poignée 16 d'une première partie 11 de corps tandis que son autre main tient la poignée 17 de l'autre partie 12 de corps pour déplacer aisément ces deux parties de corps l'une par rapport à l'autre et ainsi provoquer le déplacement de la tête de carottage 13.

Avantageusement, l'axe géométrique ou axe de carottage du dispositif de carottage 10 est celui de la tige d'actionnement 15 sur laquelle est centrée la tête de carottage 13.

Le mécanisme d'actionnement comprend également un chariot de guidage 18 supportant la tige d'actionnement 15 et reçu dans le logement interne 19 du corps principal. La tige d'actionnement 15 est avantageusement centrée.

Le chariot de guidage 18 étant solidaire de la première partie de corps mobile en translation, ce logement interne 19 définit un rail de guidage en translation de ce chariot de guidage.

Une lumière 20 s'étend à travers l'extrémité proximale du corps principal du dispositif de carottage 10, la tige d'actionnement 15 et la tête de carottage 13 pour permettre le passage d'un câble de guidage (non représenté). Ce câble est destiné au guidage, notamment l'orientation, de la tête de carottage 13 lors de son approche de la paroi ventriculaire en vue de réaliser une ouverture par carottage.

De manière avantageuse, l'extrémité distale du corps principal du dispositif de carottage 10 forme également un support d'accessoire 21.

Ce support d'accessoire 21 comporte une paroi périphérique externe tubulaire dont le diamètre extérieur est égal au diamètre de la lame de transsection 14.

Un accessoire d'assistance 22 à la pose d'une partie d'une bague d'ancrage d'une pompe cardiaque, est reçu par emboitement sur ce support d'accessoire 21. Cet accessoire d'assistance 22 est creux et comporte un fourreau s'étendant d'une portion intermédiaire de celui-ci vers son extrémité distale.

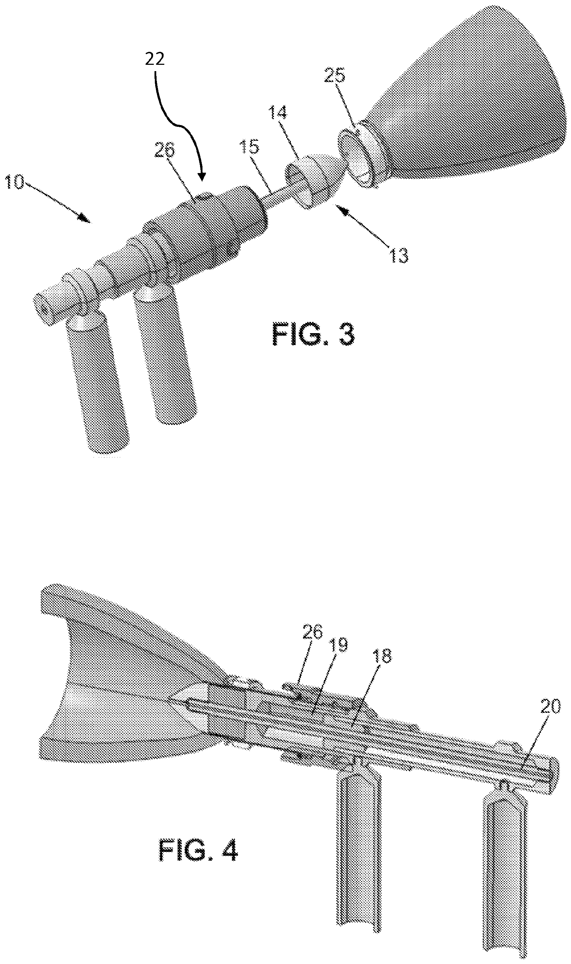

Comme représenté sur la Fig. 4, tandis que la tête de carottage 13 est encore partiellement placée dans la chambre ventriculaire, le praticien peut introduire dans l'ouverture réalisée dans la paroi ventriculaire, une partie de l'accessoire d'assistance 22, notamment son fourreau 23, en le déplaçant en translation sur le support d'accessoire 21, lequel assure un guidage linéaire.

Cet outil d'assistance étant réalisé dans un matériau rigide tel qu'en titane, son fourreau 23 maintient l'ouverture dans son extension maximale et délimite par son canal intérieur, un chemin pour le libre passage d'une partie de bague d'ancrage comprenant une portion tubulaire et à l'extrémité distale de cette dernière, une membrane auto-expansible 24 dans sa première configuration, dite déformée, dans laquelle celle-ci présente une forme tubulaire, ou essentiellement tubulaire. Cette membrane auto-expansible 24 est par exemple, réalisée en nitinol.

Une autre bride, ou seconde bride 25, de la bague d'ancrage ayant été préalablement assemblée, par exemple par suture, avec la surface extérieure de la paroi ventriculaire, l'accessoire d'assistance 22 comporte à son extrémité proximale, une tête de liaison 26 cylindrique creuse formant saillie du fourreau 23 en entourant en partie ce dernier. Cette tête de liaison 26 est destinée à être assemblée à cette seconde bride 25 pour réaliser un couplage amovible de l'accessoire avec l'ensemble seconde bride/apex du coeur. Cette seconde bride 25 est par exemple réalisée en Dacron^{®}.

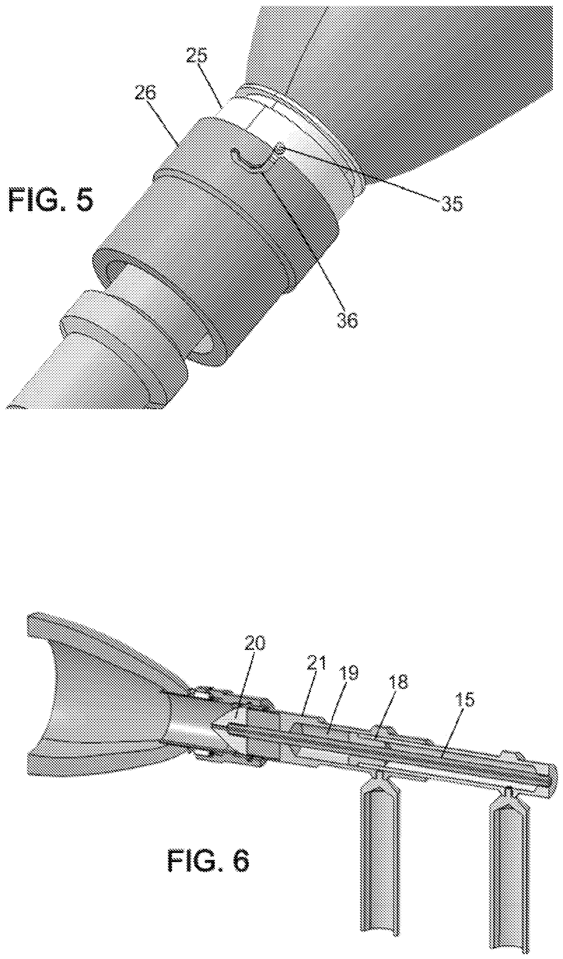

Comme illustré à la Figure 5, cette seconde bride 25 étant plaquée contre la surface extérieure de la paroi ventriculaire, elle comprend au moins un téton 35 positionné sur sa paroi latérale, la tête de liaison 26 de l'accessoire d'assistance 22 comportant pour chaque téton 35, une rainure 36 de couplage placée sur sa tranche et configurée pour recevoir ce téton correspondant. Plus précisément, chaque rainure 36 débouche à l'extrémité distale de cette tête de liaison 26 de sorte que cette dernière recouvrant partiellement la seconde bride 25 de la bague d'ancrage, chaque téton 35 est engagé dans sa rainure correspondante.

Chaque rainure 36 présente en outre une même forme incurvée de sorte que, par rotation de l'accessoire d'assistance 22 autour de l'axe de carottage, un verrouillage de cet accessoire d'assistance 22 sur la seconde bride 25 est obtenu.

Cet accessoire d'assistance 22 comporte également un élément d'étanchéité pour assurer l'étanchéité de l'assemblage tête de liaison 26/seconde bride 25 lorsque le ou les tétons ont été amenés jusqu'au fond de leur rainure correspondante sur la tête de liaison 26.

La Figure 6 est une vue en coupe du dispositif de carottage, la tête de carottage étant placée en retrait de l'ouverture réalisée dans la paroi ventriculaire, et de manière plus précise, étant placée dans le logement interne du corps du dispositif de carottage. Le fourreau 23 de l'accessoire d'assistance 22 a été introduit au travers de cette ouverture dans la chambre ventriculaire et la tête de liaison 26 est assemblée à la seconde bride 25 de la bague d'ancrage externe au coeur. Le praticien peut dès lors retirer le dispositif de carottage 10 sans risquer un déplacement de l'accessoire d'assistance 22 de l'ouverture réalisée dans la paroi ventriculaire.

Le fourreau 23 de cet accessoire d'assistance 22 permet de maintenir cette ouverture entièrement accessible pour assurer le libre passage de la partie de bague d'ancrage comprenant une portion tubulaire 27, ou corps creux, et à l'extrémité distale de cette dernière, une membrane auto-expansible 24 dans sa première configuration dite déformée.

Ce retrait du dispositif de carottage 10 entraîne la première perte d'étanchéité au niveau de la paroi ventriculaire de sorte que le praticien doit rapidement relier à cet accessoire creux, à un dispositif de poussée 28 portant cette partie de bague d'ancrage, portion tubulaire 27/membrane auto-expansible 24, pour l'introduction de son extrémité dans la chambre ventriculaire 29.

Après avoir introduit la membrane auto-expansible 24 dans cette chambre ventriculaire, et cette dernière ayant adopté sa seconde configuration dans laquelle elle définit une bride, ou collerette, s'étendant radialement à partir de la portion tubulaire 27, le praticien recule le dispositif de poussée 28 pour venir plaquer cette bride contre la face intérieure de la paroi ventriculaire 29 du coeur battant.

Ainsi, les deux brides 24, 25 étant plaquées de part et d'autre contre cette paroi ventriculaire 29, le praticien n'a plus qu'à engager un élément d'étanchéité 30 tel qu'un joint d'étanchéité torique et un moyen de serrage 31 sur le dispositif de poussée 28 et faire coulisser ces derniers le long de la surface externe de ce dispositif de poussée 28 pour venir verrouiller en position la bague d'ancrage. Ce moyen de serrage 31 vient finaliser l'assemblage de la bague d'ancrage en bloquant tout déplacement des brides 24, 25 l'une par rapport à l'autre. On obtient ainsi une liaison mécanique résistante de la bague d'ancrage et de l'apex du coeur ainsi que de son étanchéité.

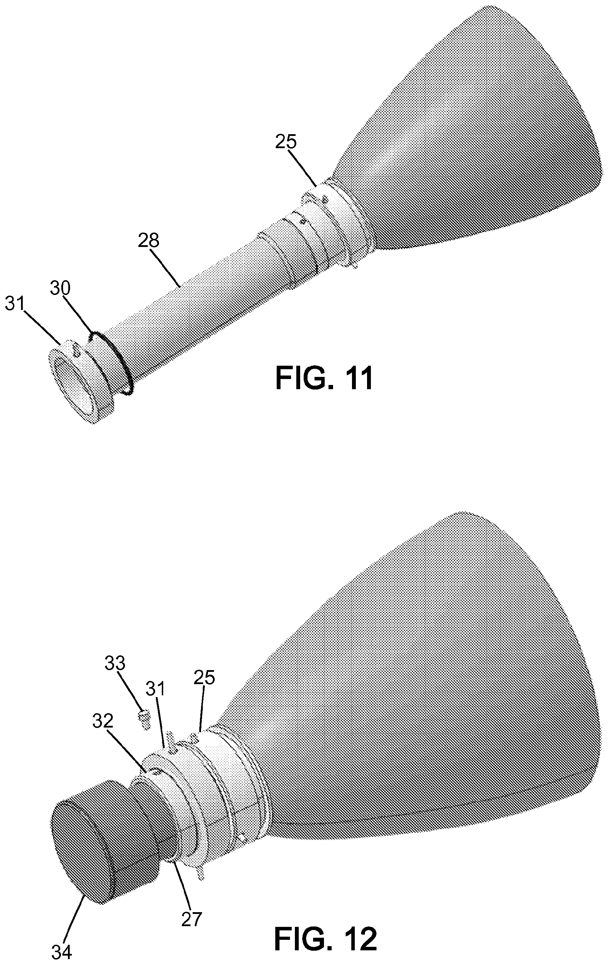

Ce moyen de serrage 31 peut être un écrou si la surface externe de la portion tubulaire 27, ou corps creux, comporte un filetage ou comme illustré à la Figure 12, une bague de serrage. Cette dernière vient presser l'élément d'étanchéité 30 contre la seconde bride 25 assemblée à la surface extérieure de la paroi ventriculaire 29.

Avantageusement, l'ensemble portion tubulaire 27/membrane auto-expansible 24 comprend un deuxième élément d'étanchéité comprenant une valve anti-reflux (non représentée) placée dans le canal interne délimité par cette portion tubulaire 27.

Cette bague de serrage 31 comporte sur sa paroi latérale un orifice 32 recevant une fixation 33 pour verrouiller en position une pompe cardiaque 34 introduite dans le canal interne délimité par le corps creux de la bague d'ancrage, l'extrémité distale de cette pompe cardiaque 34 étant engagée dans chambre ventriculaire du coeur battant.

## Revendications

1. Dispositif médical d'ancrage d'une pompe cardiaque dans une ouverture d'une paroi ventriculaire d'un coeur, comprenant :
- une première bride (25) comprenant un trou,
- un ensemble comportant un corps creux (27) à l'extrémité distale duquel est placée une membrane auto-expansible (24) entre une première configuration, dite déformée, dans laquelle ladite membrane présente une forme tubulaire et une seconde configuration, dite initiale, dans laquelle elle définit une seconde bride,
- ladite première bride (25) étant distincte dudit ensemble, ledit ensemble étant configuré pour être introduit au moins en partie au travers du trou de ladite première bride (25), et
- un moyen de serrage (31) pour assembler ladite première bride (25) et l'extrémité proximale dudit corps creux (27) lorsque cet ensemble a été inséré en partie dans ledit trou, ledit moyen de serrage (31) étant configuré pour coulisser le long dudit corps creux (27) de manière à être plaqué contre ladite première bride (25).

2. Dispositif selon la revendication 1, **caractérisé en ce que** ladite première bride (25) est réalisée dans un matériau suturable ou agrafable.

3. Dispositif selon la revendication 1, **caractérisé en ce que** ladite première bride (25) comportant deux faces d'extrémités reliées entre elles par une paroi latérale, sa face d'extrémité destinée à venir en aboutement contre la surface externe de ladite paroi ventriculaire, comprend une rainure circulaire en communication avec un orifice débouchant sur sa paroi latérale.

4. Dispositif selon l'une quelconque des revendications précédentes, **caractérisé en ce qu'**il comprend un premier élément d'étanchéité (30) destiné à être placé entre ledit moyen de serrage (31) et ladite première bride (25).

5. Dispositif selon l'une quelconque des revendications précédentes, **caractérisé en ce qu'**il comprend un deuxième élément d'étanchéité comprenant une valve anti-reflux placée dans le canal interne délimité par ledit corps creux (27).

6. Dispositif selon l'une quelconque des revendications précédentes, **caractérisé en ce que** ledit corps creux (27) a une longueur supérieure à la somme de l'épaisseur de la paroi ventriculaire et de ladite première bride (25).

7. Dispositif selon l'une quelconque des revendications précédentes, **caractérisé en ce que** ledit moyen de serrage (31) est configuré pour réaliser un couplage réversible, ou temporaire, de ladite première bride (25) et de l'extrémité proximale dudit corps creux (27).

8. Dispositif selon l'une quelconque des revendications précédentes, **caractérisé en ce que** ledit moyen de serrage (31) est un écrou.

9. Dispositif selon la revendication 8, **caractérisé en ce que** cet écrou présentant un filetage interne, la surface externe d'une partie du corps creux (27), en particulier son extrémité proximale, comporte un filetage complémentaire du filetage de l'écrou pour assurer l'engagement et le déplacement de l'écrou le long de ladite partie de surface externe dudit corps creux (27).

10. Dispositif selon l'une quelconque des revendications précédentes, **caractérisé en ce que** l'extrémité proximale du corps creux (27) comporte un alésage pour le vissage ou le passage d'une fixation de manière à bloquer en position une pompe cardiaque introduite dans ce corps creux (27).

## Patentansprüche

1. Medizinische Vorrichtung zur Verankerung einer Herzpumpe in einer Öffnung einer ventrikulären Wand eines Herzens, umfassend:
- einem ersten Flansch (25), der ein Loch umfasst,
- eine Anordnung, umfassend einen Hohlkörper (27), an dessen distalem Ende eine selbstexpandierende Membran (24) platziert ist, zwischen einer ersten Konfiguration, der sogenannten verformten Konfiguration, in der die Membran eine rohrförmige Form aufweist, und einer zweiten Konfiguration, der sogenannten Anfangskonfiguration, in der sie einen zweiten Flansch begrenzt,
- wobei der erste Flansch (25) von der Anordnung getrennt ist, wobei die Anordnung dazu ausgebildet ist, wenigstens teilweise durch das Loch des ersten Flansches (25) eingeführt zu werden, und
- ein Klemmmittel (31) zum Zusammenfügen des ersten Flansches (25) und des proximalen Endes des Hohlkörpers (27), wenn diese Anordnung teilweise in das Loch eingeführt worden ist, wobei das Klemmmittel (31) dazu ausgebildet ist, entlang des Hohlkörpers (27) zu gleiten, um gegen den ersten Flansch (25) gedrückt zu werden.

2. Vorrichtung nach Anspruch 1, **dadurch gekennzeichnet, dass** der erste Flansch (25) aus einem nähbaren oder klammerbaren Material hergestellt ist.

3. Vorrichtung nach Anspruch 1, **dadurch gekennzeichnet, dass** der erste Flansch (25) zwei Endflächen aufweist, die durch eine Seitenwand miteinander verbunden sind, wobei seine Endfläche, die dazu bestimmt ist, an der Außenfläche der Ventrikelwand anzuliegen, eine kreisförmige Nut aufweist, die mit einer Öffnung in Verbindung steht, die an seiner Seitenwand mündet.

4. Vorrichtung nach einem der vorhergehenden Ansprüche, **dadurch gekennzeichnet, dass** sie ein erstes Dichtungselement (30) umfasst, das dazu bestimmt ist, zwischen dem Klemmmittel (31) und dem ersten Flansch (25) angeordnet zu werden.

5. Vorrichtung nach einem der vorhergehenden Ansprüche, **dadurch gekennzeichnet, dass** sie ein zweites Dichtungselement umfasst, das ein Rückschlagventil umfasst, das in dem von dem Hohlkörper (27) begrenzten inneren Kanal angeordnet ist.

6. Vorrichtung nach einem der vorhergehenden Ansprüche, **dadurch gekennzeichnet, dass** der Hohlkörper (27) eine Länge aufweist, die größer ist als die Summe der Dicke der ventrikulären Wand und des ersten Flansches (25).

7. Vorrichtung nach einem der vorhergehenden Ansprüche, **dadurch gekennzeichnet, dass** das Klemmmittel (31) dazu ausgebildet ist, eine reversible, oder temporäre Kopplung des ersten Flansches (25) und des proximalen Endes des Hohlkörpers (27) zu bewirken.

8. Vorrichtung nach einem der vorhergehenden Ansprüche, **dadurch gekennzeichnet, dass** das Klemmmittel (31) eine Mutter ist.

9. Vorrichtung nach Anspruch 8, **dadurch gekennzeichnet, dass** diese Mutter ein Innengewinde aufweist, wobei die Außenfläche eines Teils des Hohlkörpers (27), insbesondere sein proximales Ende, ein Gewinde aufweist, das komplementär zum Gewinde der Mutter ist, um den Eingriff und die Bewegung der Mutter entlang des Teils der Außenfläche des Hohlkörpers (27) zu gewährleisten.

10. Vorrichtung nach einem der vorhergehenden Ansprüche, **dadurch gekennzeichnet, dass** das proximale Ende des Hohlkörpers (27) eine Bohrung zum Einschrauben oder Durchführen einer Befestigung aufweist, um eine in diesen Hohlkörper (27) eingeführte Herzpumpe in ihrer Position zu blockieren.

## Claims

1. A medical device for anchoring a heart pump in an opening of a ventricular wall of a heart, comprising:
- a first flange (25) comprising a hole,
- an assembly including a hollow body (27), at the distal end of which a membrane (24) is placed which is self-expandable between a first configuration, so-called the deformed configuration, in which said membrane has a tubular shape, and a second configuration, so-called the initial configuration, in which it defines a second flange,
- said first flange (25) being distinct from said assembly, said assembly being configured to be inserted at least partially throughout the hole of said first flange (25), and
- a clamping means (31) for assembling said first flange (25) and the proximal end of said hollow body (27) when this assembly has been partially inserted into said hole, said clamping means (31) being configured to slide along said hollow body (27) so as to be pressed against said first flange (25).

2. The device according to claim 1, **characterised in that** said first flange (25) is made of a material that can be stitched or stapled.

3. The device according to claim 1, **characterised in that** said first flange (25) including two end faces connected to each other by a lateral wall, its end face intended to butt against the outer surface of said ventricular wall, comprises a circular groove in communication with an orifice opening onto its lateral wall.

4. The device according to any one of the preceding claims, **characterised in that** it comprises a first sealing element (30) intended to be placed between said clamping means (31) and said first flange (25).

5. The device according to any one of the preceding claims, **characterised in that** it comprises a second sealing element comprising an anti-reflux valve placed in the inner channel delimited by said hollow body (27).

6. The device according to any one of the preceding claims, **characterised in that** said hollow body (27) has a length greater than the sum of the thickness of the ventricular wall and of said first flange (25).

7. The device according to any one of the preceding claims, **characterised in that** said clamping means (31) is configured to perform a reversible or temporary coupling of said first flange (25) and of the proximal end of said hollow body (27).

8. The device according to any one of the preceding claims, **characterised in that** said clamping means (31) is a nut.

9. The device according to claim 8, **characterised in that**, this nut having an internal thread, the outer surface of a portion of the hollow body (27), in particular its proximal end, includes a thread matching the thread of the nut to ensure the engagement and the movement of the nut along said outer surface portion of said hollow body (27).

10. The device according to any one of the preceding claims, **characterised in that** the proximal end of the hollow body (27) includes a bore for screwing or passage of a fastener, so as to block in position a heart pump inserted into this hollow body (27).
